# NEUE EUROPÄISCHE PATENTSCHRIFT

(11) **EP 2 705 022 B2**
(45) Veröffentlichungstag und Bekanntmachung des Hinweises auf die Entscheidung über den Einspruch: **28.04.2021**
(45) Hinweis auf die Patenterteilung: 06.12.2017
(21) Anmeldenummer: 12716431.7
(22) Anmeldetag: 25.04.2012
(51) Int. Cl.: C07C 227/42, C07C 229/04

(54) **VERFAHREN ZUR HERSTELLUNG EINES KRISTALLINEN L-MGDA-TRIALKALIMETALLSALZES**
PROCESS FOR PREPARING A CRYSTALLINE L-MGDA TRI-ALKALI METAL SALT
PROCÉDÉ DE PRODUCTION D'UN SEL DE MÉTAL TRIALCALIN L-MGDA-CRISTALLIN

(30) Priorität: 03.05.2011 EP 11164601
(43) Veröffentlichungstag der Anmeldung: 12.03.2014
(73) Patentinhaber: BASF SE, 67056 Ludwigshafen am Rhein (DE)
(72) Erfinder: OFTRING, Alfred, 67098 Bad Dürkheim (DE); WEBER, Maxim, 67117 Limburgerhof (DE); BRAUN, Gerold, 67071 Ludwigshafen (DE); LAUTERBACH, Arnulf, 67067 Ludwigshafen (DE)
(74) Vertreter: BASF IP Association
(86) Internationale Anmeldenummer: PCT/EP2012/057508
(87) Internationale Veröffentlichungsnummer: WO 2012/150155

(56) Entgegenhaltungen:
- WO-A1-94/29421
- NORIO KOINE ET AL.,: "Preparation and Circular Dichroism of trans (N) and cis(N) Isomers of (L- or D-Alaninate-N,N-diacetato) (amino-acidato)-cobaltate(III) Complexes", BULLETIN OF THE CHEMICAL SOCIETY OF JAPAN, Bd. 43, 1970, Seiten 1737-1743, XP002682068,

## Beschreibung

Komplexbildner für Erdalkali- und Schwermetallionen werden in weiten Bereichen der Industrie, zum Beispiel in der Wasch- und Reinigungsmittelindustrie oder bei der Behandlung von Metalloberflächen, eingesetzt. Üblicherweise werden sie in wässriger Lösung synthetisiert. Für bestimmte Anwendungsfälle werden sie in fester Form benötigt.

Übliche Verfahren zur Herstellung von Feststoffen aus wässrigen Lösungen sind insbesondere Kristallisations- und Sprühtrocknungsverfahren. Bekannt ist, dass kristalliner Feststoff, wie er beispielsweise bei Verdampfungs- oder Kühlungskristallisation anfällt, Kristallwasser enthalten kann und unter Umgebungsbedingungen meist weniger hygroskopisch oder lagerstabil als amorpher Feststoff ist. Durch Sprühtrocknungsverfahren, z. B. im Sprühturm oder im Sprühwirbelbett, dagegen wird der Feststoff amorph erhalten. In dieser Form ist der Feststoff oft stark hygroskopisch und verliert bei offener Lagerung innerhalb kurzer Zeit die Rieselfähigkeit, was die Weiterverarbeitbarkeit, z. B. in Tablettenpressen etc., stark erschwert.

Methylglycindiessigsäure, im Folgenden abgekürzt als MGDA bezeichnet, auch als α-Alanin-Diessigsäure bekannt (α-ADA), ist ein starker, biologisch leicht abbaubarer Komplexbildner für verschiedene technische Anwendungen und ist z. B. aus der WO-A 94/29421 bekannt.

In Form des racemischen Tri-Natriumsalz-Gemisches ist die Kristallisation durch die unsymmetrische Molekülform stark gehemmt, was zu entsprechend aufwändigen und unwirtschaftlichen Massenkristallisationsverfahren (Verdampfungs- und Kühlungskristallisation) führt.

Die EP-A 0 8 45 456 beschreibt ein Verfahren zur Herstellung von MGDA-Na3-Pulvern mit erhöhtem Kristallinitätsgrad, wobei insbesondere von Ausgangsmassen mit Wasseranteilen von 10-30 % ausgegangen wird und bevorzugt Kristallisationskeime zugegeben werden. Dieses Verfahren führt zu überwiegend kristallinen Pulvern, erfordert aber wegen der zähen und pastösen Phasen während der Herstellung den Einsatz von aufwändigen und teuren Mischer-Kneter-Apparaturen, um die Umwandlung in die kristallinen Modifikationen zu gewährleisten.

Es war demgegenüber Aufgabe der Erfindung, ein technisch einfaches Verfahren zur Herstellung von kristallinen MGDA-Trialkalimetallsalzen zur Verfügung zu stellen.

Die Lösung besteht in einem Verfahren zur Herstellung eines kristallinen L-MGDA-Trinatriumsalzes durch Kristallisation aus einer wässrigen Lösung desselben, die man erhält indem man von L-α-Alanin ausgeht, das man durch Umsetzung mit Formaldehyd und Blausäure zu L-α-Alanin-N,N-Diacetonitril gemäß Strecker-Synthese umsetzt und anschließend das L-α-Alanin-N,N-Diacetonitril zum L-MGDA-Trinatriumsalz alkalisch verseift, wobei bei der alkalischen Verseifung eine Temperatur von 150 °c nicht überschritten wird.

Es wurde überraschend gefunden, dass die Kristallisation von L-MGDA-Trinatriumsalz mit deutlichen Vorteilen im Vergleich zum entsprechenden Racemat abläuft, mit entsprechenden anwendungstechnischen Vorteilen des Kristallisats.

Erfindungsgemäß wird von dem L-Enantiomeren des α-Alanins ausgegangen, das durch Strecker-Synthese mit Formaldehyd und Blausäure zum L-α-Alanin-N,N-Diacetonitril, im Folgenden abgekürzt als L-ADAN bezeichnet, umgesetzt wird. Das L-ADAN wird anschließend mit einer Base zum L-MGDA-Trinatriumsalz verseift.

Die Strecker-Synthese kann in jeder bekannten Weise durchgeführt werden, zum Beispiel gemäß dem in der WO-A 94/29421 offenbarten Verfahren.

Unabhängig von der konkreten Durchführung der Strecker-Synthese ist es wesentlich für die vorliegende Erfindung, dass die alkalische Verseifung des L-ADAN stets unter Bedingungen erfolgt, die gewährleisten, dass das ausgehend von L-α-Alanin erhaltene L-MGDA-Trinatriumsalz nicht racemisiert. Dafür ist es ausreichend sicherzustellen, dass die Temperatur bei der alkalischen Verseifung 150 °C nicht überschreitet.

Vorteilhaft soll die Temperatur der alkalischen Verseifung 130 °C nicht überschreiten. Besonders vorteilhaft ist es, die alkalische Verseifung bei einer Temperatur durchzuführen, die 110 °C nicht überschreitet.

Die alkalische Verseifung des L-ADAN kann mit Natronlauge durchgeführt werden.

Nach dem obigen Verfahren der Strecker-Synthese mit anschließender alkalischer Hydrolyse werden bevorzugt L-MGDA-Trinatriumsalz-Lösungen mit einem Gehalt an L-MGDA-Trinatriumsalz von mindestens 50 Gew.-%, bezogen auf das Gesamtgewicht der Lösung, oder auch von mindestens 40 Gew.-% MGDA-Trinatriumsalz, bezogen auf das Gesamtgewicht der Lösung, erhalten. Es wurde gefunden, dass sich aus diesen Lösungen überraschend einfach das entsprechende L-MGDA-Trinatriumsalz auskristallisieren lässt.

Hierzu kann vorteilhaft eine Verdampfungskristallisation eingesetzt werden.

Die Verdampfungskristallisation kann vorteilhaft kontinuierlich durchgeführt werden.

In einer anderen Ausführungsvariante kann die Verdampfungskristallisation diskontinuierlich durchgeführt werden.

Bevorzugte Verfahrensparameter sowohl für die kontinuierliche wie auch die diskontinuierliche Verdampfungskristallisation sind Temperaturen im Bereich von 50 bis 130 °C, bevorzugt im Bereich von 70 bis 120 °C, weiter bevorzugt im Bereich von 80 bis 100 °C.

Vorteilhaft wird die kontinuierliche oder diskontinuierliche Verdampfungskristallisation während einer Dauer von 1 bis 24 Stunden, bevorzugt von 5 bis 24 Stunden, weiter bevorzugt von 5 bis 10 Stunden, durchgeführt.

Die Verdampfungsgeschwindigkeit, definiert durch die Verdampfungsdauer und den Kristallgehalt am Ende der Verdampfungskristallisation liegt für die kontinuierliche Verdampfungskristallisation insbesondere bei 1 bis 50 %, bevorzugt bei 10 bis 30 % und weiter bevorzugt bei 10 bis 20 %; für die diskontinuierliche Verdampfungskristallisation insbesondere bei 1 bis 60 %, bevorzugt 10 bis 40 %, weiter bevorzugt 10 bis 20 %.

In einer anderen Ausführungsform kann die Kristallisation durch Kühlungskristallisation durchgeführt werden.

Das Verfahren ist nicht eingeschränkt bezüglich der Apparate, in denen die Kristallisation durchgeführt wird.

Die erfindungsgemäße Kristallisation der L-Enantiomeren von MGDA-Trinatriumsalz aus dessen wässrigen Lösungen weist gegenüber Kristallisationen aus dem entsprechenden D,L-Racemat erhebliche Vorteile auf: Die Kristalle entstehen in Anwesenheit von Keimen allmählich, was die Kontrolle der Kristallisation erleichtert. Dagegen kristallisiert das Racemat spontan bei hoher Übersättigung, wobei Animpfen keine deutliche Verbesserung bringt.

Gegenüber einer Kristallisation aus der Lösung des Racemats ist bei der erfindungsgemäßen Kristallisation aus der Lösung des L-Enantiomeren eine bessere Kontrolle des Feststoffgehaltes in der Suspension möglich; dadurch ist die Wahrscheinlichkeit von Fehlchargen geringer, der Kristallisationsapparat setzt sich nicht zu.

Gegenüber einer Kristallisation aus einer Racematlösung werden nach dem erfindungsgemäßen Verfahren Kristalle mit einer deutlich besseren Morphologie erhalten, und zwar dreidimensionale Partikel gegenüber Nadeln. Daraus resultiert eine deutlich verbesserte Rührbarkeit der Suspensionen sowie wesentlich höhere Raum-Zeit-Ausbeuten. So ist beispielsweise eine Suspension mit 12 Gew.-% D,L-MGDA-Trinatriumsalznadeln praktisch fest, während eine solche mit 30 Gew.-% L-MGDA-Trinatriumsalzkristallen noch gut rührbar ist. Weiterhin ist die Zentrifugierbarkeit besser. Aufgrund der kleineren spezifischen Oberfläche ist die Reinheit höher. Der Bruch bei dreidimensionalen Partikeln ist deutlich niedriger als bei Nadeln, und dadurch auch der Feinanteil, auch die Lager- und Transporteigenschaften sind besser. Insbesondere sind die im erfindungsgemäßen Verfahren erhaltenen Kristalle im Vergleich zu einem beispielsweise durch Sprühtrocknung erhaltenen Pulver nicht hygroskopisch.

Die Erfindung wird im Folgenden anhand von Ausführungsbeispielen und einer Zeichnung näher erläutert.

Es wurden jeweils ca. 40%ige Ausgangslösungen von D,L-MGDA-Trinatriumsalz bzw. L-MGDA-Trinatriumsalz, ausgehend von D,L-α-Alanin bzw. L-α-Alanin wie folgt hergestellt:
Zu einer Lösung von 178 g (2,0 mol) α-Alanin in 910 g Wasser (ca. 18%ig) wurde bei ca. 40 °C unter Kühlen innerhalb von ca. 60 Minuten 203 g 30%iger Formaldehyd (2,03 mol) dosiert. Zur resultierenden Lösung wurden anschließend bei ca. 40 °C parallel innerhalb von 60 Minuten 203 g 30%iger Formaldehyd (2,03 mol) und 109,6 g Blausäure (4,06 mol) unter Kühlen dosiert. Man ließ 1 Stunde bei 40 °C nachreagieren. Diese Lösung wurde anschließend bei ca. 30 °C innerhalb von ca. 1 Stunde zu 496 g 50%iger Natronlauge (6,20 mol) dosiert. Nach zweistündigem Nachrühren bei 30 °C wurde auf 95-102 °C erhöht und innerhalb von ca. 4 Stunden der Umsatz vervollständigt, unter gleichzeitigem Abstrippen des gebildeten Ammoniaks und von Wasser. Man erhielt 1330 g einer ca. 40%igen MGDA-Tri-Natriumsalz-Lösung (MGDA-Na3).
Ausbeute: 98,2, %; NTA-Na3-Gehalt: 0,06 % (Bestimmung mit HPLC)
**Vergleichsbeispiel:** Diskontinuierliche Verdampfungskristallisation von D,L-MGDA-Trinatriumsalz

In einem 3-L-Rührwerkskristallisator wurden 1200 g einer 40%igen D,L-MGDA-Trinatriumsalz-Lösung vorgelegt und durch die Verdampfung zur Sättigung bei 80 °C gebracht (46 % für D,L-MGDA). Danach wurde die Lösung mit ca. 0,5 g der Kristalle aus einem Vorversuch geimpft. Anschließend folgte die Eindampfung bei 80 °C mit 50 g/h. Es fand keine Kristallisation statt, bis es nach dem Abdampfen von 95 g zu einem massiven Keimschauer kam und der Kristallisator komplett durchkristallisiert war. Die so entstandene Suspension ließ sich nicht mehr rühren oder filtrieren (theoretischer Feststoffanteil in der Suspension am Ende 9 %). Die Kristalle hatten die Form feiner Nadeln mit einer Länge unter 100 µm.

### Beispiel 1: Diskontinuierliche Verdampfungskristallisation von L-MGDA-Natriumsalz

In einem 3-L-Rührwerkkristallisator wurden 2154 g einer 40%igen L-MGDA-Natriumsalz-Lösung vorgelegt und bis zu einer Konzentration von 58 % eingedampft, was einer leichten Übersättigung bei 80 °C entspricht. Danach wurde die Lösung mit 1 % der Kristalle bezogen auf den Feststoffgehalt der Lösung geimpft. Die Kristalle wurden in einem Vorversuch erzeugt. Anschließend folgte die Eindampfung bei 80 °C mit 52 g/h. Während der Eindampfung entstanden gut definierte kompakte Kristalle mit der Größe von ca. 20-100 µm. Die Suspension ließ sich bis zum Ende des Versuchs gut rühren (Feststoffanteil in der Suspension am Ende 36 %). Der während der Filtration ermittelte Filterwiderstand lag bei 2,25 x 10¹³ mPas/m², das heißt die Kristalle lassen sich durch Filtration abtrennen.

### Beispiel 2: Kontinuierliche Verdampfungskristallisation von L-MGDA-Natriumsalz

Zum Anfahren wurden in einem 1-L-Rührwerckristallisator 1280 g der übersättigten 65%igen L-MGDA-Natriumsalz-Lösung vorgelegt. Danach wurde die Lösung angeimpft, um eine Suspension herzustellen. Anschließend folgte die kontinuierliche Verdampfungskristallisation bei 90 °C mit 454 g/h Feed und 204 g/h Abdampfrate. Dies entspricht einer Verweilzeit von 5 h und einem theoretischen Feststoffanteil in der Suspension von 41 %. Während der Eindampfung entstanden gut definierte kompakte Kristalle mit der Größe von ca. 10-500 µm. Die Suspension ließ sich zum Ende des Versuchs gut rühren. Der während der Filtration ermittelte Filterwiderstand lag bei 12,7 X 10¹³ mPas/m², das heißt die Kristalle lassen sich durch Filtration abtrennen.

In der Zeichnung zeigen im Einzelnen:
- Figur 1:: eine Mikroskopaufnahme der nach dem Vergleichsbeispiel erhaltenen Nadeln,
- Figur 2:: eine Mikroskopaufnahme der nach Beispiel 1 erhaltenen Kristalle und
- Figur 3:: eine Mikroskopaufnahme der nach Beispiel 2 erhaltenen Kristalle.

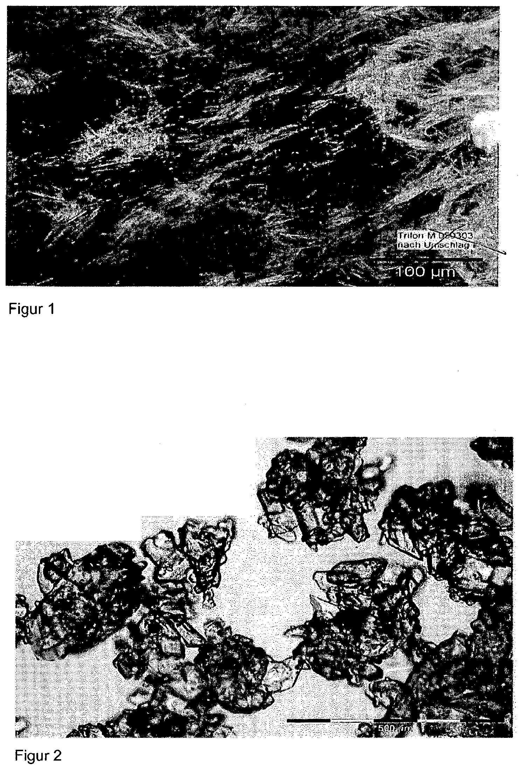

In der Figur 1 ist zu erkennen, dass feine Nadeln mit einer Länge unter 100 µm erhalten wurden.

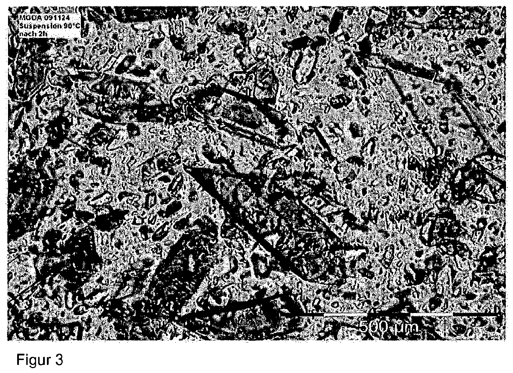

Die Figuren 2 und 3 dagegen zeigen deutliche Kristalle.

## Patentansprüche

1. Verfahren zur Herstellung eines kristallinen L-MGDA-Trinatriumsalzes durch Kristallisation aus einer wässrigen Lösung desselben, die man erhält, indem man von L-α-Alanin ausgeht, das man durch Umsetzung mit Formaldehyd und Blausäure zu L-α-Alanin-N,N-Diacetonitril gemäß Strecker-Synthese umsetzt und anschließend das L-α-Alanin-N,N-Diacetonitril zum L-MGDA-Trinatriumsalz alkalisch verseift, wobei bei der alkalischen Verseifung eine Temperatur von 150 °C nicht überschritten wird.

2. Verfahren nach Anspruch 1, **dadurch gekennzeichnet, dass** bei der alkalischen Verseifung eine Temperatur von 130 °C nicht überschritten wird.

3. Verfahren nach Anspruch 1, **dadurch gekennzeichnet, dass** bei der alkalischen Verseifung eine Temperatur von 110 °C nicht überschritten wird.

4. Verfahren nach einem der Ansprüche 1 bis 3, **dadurch gekennzeichnet, dass** die Kristallisation als Verdampfungskristallisation durchgeführt wird.

5. Verfahren nach einem der Ansprüche 1 bis 3, **dadurch gekennzeichnet, dass** die Kristallisation als Kühlungskristallisation durchgeführt wird.

6. Verfahren nach Anspruch 4, **dadurch gekennzeichnet, dass** die Verdampfungskristallisation bei einer Temperatur im Bereich von 50-130 °C durchgeführt wird.

7. Verfahren nach Anspruch 4 oder 6, **dadurch gekennzeichnet, dass** die Verdampfungskristallisation während einer Dauer von 1 bis 24 Stunden durchgeführt wird.

8. Verfahren nach einem der Ansprüche 4, 6 oder 7, **dadurch gekennzeichnet, dass** die Verdampfungskristallisation diskontinuierlich durchgeführt wird.

9. Verfahren nach einem der Ansprüche 4, 6 oder 7, **dadurch gekennzeichnet, dass** die Verdampfungskristallisation kontinuierlich durchgeführt wird.

10. Verfahren nach einem der Ansprüche 1 bis 9, **dadurch gekennzeichnet, dass** die Kristallisation in einem Rührwerkkristallisator durchgeführt wird.

## Claims

1. A process for the preparation of a crystalline L-MGDA trisodium salt by crystallization from an aqueous solution thereof which is obtained by starting from L-α-alanine which is reacted by reaction with formaldehyde and hydrocyanic acid to give L-α-alanine-N,N-diacetonitrile according to Strecker synthesis and the subsequent subjection of L-α-alanine-N,N-diacetonitrile to alkaline saponification to give the L-MGDA trisodium salt, wherein a temperature of 150°C is not exceeded during the alkaline saponification.

2. The process according to claim 1, wherein a temperature of 130°C is not exceeded during the alkaline saponification.

3. The process according to claim 1, wherein a temperature of 110°C is not exceeded during the alkaline saponification.

4. The process according to any one of claims 1 to 3, wherein the crystallization is carried out as evaporation crystallization.

5. The process according to any one of claims 1 to 3, wherein the crystallization is carried out as cooling crystallization.

6. The process according to claim 4, wherein the evaporation crystallization is carried out at a temperature in the range from 50-130°C.

7. The process according to claim 4 or 6, wherein the evaporation crystallization is carried out over a period from 1 to 24 hours.

8. The process according to any one of claims 4, 6 or 7, wherein the evaporation crystallization is carried out discontinuously.

9. The process according to any one of claims 4, 6 or 7, wherein the evaporation crystallization is carried out continuously.

10. The process according to any one of claims 1 to 9, wherein the crystallization is carried out in a crystallizer with agitator.

## Revendications

1. Procédé de fabrication d'un sel de trisodium de L-MGDA cristallin par cristallisation à partir d'une solution aqueuse de celui-ci, qui est obtenue en partant de L-α-alanine, qui est transformée par mise en réaction avec du formaldéhyde et de l'acide cyanhydrique en L-α-alanine-N,N-diacétonitrile selon la synthèse de Strecker, puis le L-α-alanine-N,N-diacétonitrile est saponifié alcaliniquement en le sel de trisodium de L-MGDA, une température de 150 °C n'étant pas dépassée lors de la saponification alcaline.

2. Procédé selon la revendication 1, **caractérisé en ce qu'**une température de 130 °C n'est pas dépassée lors de la saponification alcaline.

3. Procédé selon la revendication 1, **caractérisé en ce qu'**une température de 110 °C n'est pas dépassée lors de la saponification alcaline.

4. Procédé selon l'une quelconque des revendications 1 à 3, **caractérisé en ce que** la cristallisation est réalisée sous la forme d'une cristallisation par évaporation.

5. Procédé selon l'une quelconque des revendications 1 à 3, **caractérisé en ce que** la cristallisation est réalisée sous la forme d'une cristallisation par refroidissement.

6. Procédé selon la revendication 4, **caractérisé en ce que** la cristallisation par évaporation est réalisée à une température dans la plage allant de 50 à 130 °C.

7. Procédé selon la revendication 4 ou 6, **caractérisé en ce que** la cristallisation par évaporation est réalisée pendant une durée de 1 à 24 heures.

8. Procédé selon l'une quelconque des revendications 4, 6 ou 7, **caractérisé en ce que** la cristallisation par évaporation est réalisée de manière discontinue.

9. Procédé selon l'une quelconque des revendications 4, 6 ou 7, **caractérisé en ce que** la cristallisation par évaporation est réalisée de manière continue.

10. Procédé selon l'une quelconque des revendications 1 à 9, **caractérisé en ce que** la cristallisation est réalisée dans un cristallisateur à agitateur.
